# EUROPEAN PATENT SPECIFICATION

(11) **EP 2 034 910 B1**
(45) Date of publication and mention of the grant of the patent: **15.02.2012**
(21) Application number: 07796648.9
(22) Date of filing: 02.07.2007
(51) Int. Cl.: A61B 17/28, A61B 18/14, A61B 17/00

(54) **SURGICAL INSTRUMENT WITH DETACHABLE TOOL ASSEMBLY**
CHIRURGISCHES INSTRUMENT MIT ABNEHMBARER WERKZEUGANORDNUNG
INSTRUMENT CHIRURGICAL PRÉSENTANT UN ENSEMBLE D'OUTIL DÉTACHABLE

(30) Priority: 30.06.2006 US 818159 P
(43) Date of publication of application: 18.03.2009
(73) Proprietor: Bovie Medical Corporation, Clearwater, FL 33760 (US)
(72) Inventor: LIVNEH, Steve, Amherstburg, ON N9V 1X3 (CA)
(74) Representative: McDonough, Jonathan
(86) International application number: PCT/US2007/015358
(87) International publication number: WO 2008/005433

(56) References cited:
- WO-A-98/01080
- DE-A1- 19 520 717
- DE-U1- 29 503 626
- US-A1- 2004 260 201

## Description

### BACKGROUND OF THE INVENTION

### 1. Field of the Invention

The invention relates to a surgical instrument having a detachable tool assembly.

2. Description of the Related Art

Surgical instruments, such as forceps, are well known in the art for use in surgery and other medical procedures. Commonly, forceps, such as Kelly forceps, have a two piece construction like scissors, but are utilized to grip instead of cut. These common forceps includes a pair of handles attached to a pair of jaws.

During advanced endoscopic and arthroscopic surgeries, a surgeon may require several different styles and sizes of jaws depending on a wide variety of factors. To save on disposal and sterilization costs, medical device suppliers began offering surgical instruments with detachable tool assemblies.

One such instrument is disclosed in U.S. Patent No. 5,893,875 (the '875 patent) to O'Connor et al. The instrument of the '875 patent includes a handle connected to a shaft. The instrument also includes a detachable tool;, such as scissors. A first sleeve connected to the tool mates with a second sleeve connected to the shaft. Disconnection of the tool from the shaft may be clumsy and requires two-handed rotation of components.
US 2004/260201 A1 (Mueller Richard L) discloses a cytology brush suitable for conducting a brushing biopsy within a mammary duct of a human patient. The cytology brush comprises a handle and a detachable or releasable brush. After a brushing biopsy the brush end can be released and retained in the collection vessel.

Therefore, there remains an opportunity for a surgical instrument with an easily detachable tool assembly that can be operated with a single hand.

### SUMMARY OF THE INVENTION AND ADVANTAGES

The present invention provides a surgical instrument comprising:
a shaft having a proximal end and a distal end;
a housing surrounding at least a portion of said shaft and defining a central opening and a cavity wherein said cavity has a cross-sectional width greater than a cross-sectional width of said central opening;
at least one handle operatively connected to said proximal end of said shaft for longitudinally actuating said shaft within said housing;
a detachable tool assembly including a connection rod having a proximal end and a distal end and at least one tool operatively connected to said connection rod; and
a collet disposed within said housing and detachably coupling said shaft and said connection rod;
said collet including a base attached to said distal end of said shaft and a plurality of expandable fingers extending from said base such that said fingers capture said proximal end of said connection rod when in a collapsed position and release said proximal end of said connection rod when in an expanded position; and
said collet is moveable within said housing between an ejected position and at least one operating position as said shaft moves longitudinally through said housing wherein said fingers are collapsed within said central opening and capture said ball-shaped proximal end of said connection rod in said at least one operating position and said fingers flare away from one another in said cavity to release said connection rod in said ejected position.

The surgical instrument of the present invention allows for quick detachment of the detachable tool assembly merely by actuating the handle. This detachment can be formed by an operator of the instrument (e.g., a surgeon or assistant) with a single hand, thus allowing the operator's other hand to be free to perform other tasks. Furthermore, the operator need not remove his hand from the handles of the instrument to detach the tool assembly. This prevents potential contamination by handling of the tool assembly.

### BRIEF DESCRIPTION OF THE DRAWINGS

Other advantages of the present invention will be readily appreciated, as the same becomes better understood by reference to the following detailed description when considered in connection with the accompanying drawings wherein:

Figure 1 is a perspective view of a first embodiment of a surgical instrument with a detachable tool assembly having a pair of jaws and operable by a pair of handles;

Figure 2 is a cross-sectional view of the first embodiment of the surgical instrument with the jaws and handles in an open position;

Figure 3 is a cross-sectional view of the first embodiment of the surgical instrument with the jaws and handles in a closed position;

Figure 4 is a cross-sectional view of the first embodiment of the surgical instrument with the jaws and handles in an ejected position;

Figure 5 is a perspective view of the detachable tool assembly of the first embodiment detached from a housing;

Figure 6 is a cross-sectional view of the jaws of the detachable tool assembly of the first embodiment detached from the housing;

Figure 7 is an exploded view of a portion of the first embodiment of the surgical instrument showing the detachable tool assembly, a clevis component, the housing, and a shaft;

Figure 8 is a cross-sectional view of the jaws of the detachable tool assembly of the first embodiment in the closed position;

Figure 9 is a perspective view of a collet with fingers in a compressed position;

Figure 10 is a perspective view of the collet with fingers in a flared position;

Figure 11 is a perspective view of a first configuration of a coupling between the collet and the housing;

Figure 12 is a cross-sectional view of the housing in the first configuration;

Figure 13 is a perspective view of the detachable tool assembly of the first embodiment in a second configuration;

Figure 14 is a cross-sectional view of the detachable tool assembly connected to the housing in the first embodiment and the second configuration showing the jaws in the open position;

Figure 15 is a cross-sectional view of the detachable tool assembly connected to the housing in the first embodiment and the second configuration showing the jaws in the closed position;

Figure 16 is a cross-sectional view of the detachable tool assembly detached from the housing in the first embodiment and the second configuration;

Figure 17 is a perspective view of the second configuration of the coupling between the collet and the housing;

Figure 18 is a cross-sectional view of the housing in the second configuration;

Figure 19 is a perspective view of the detachable tool assembly and housing of the first embodiment in a third configuration;

Figure 20 is a cross-sectional view of the detachable tool assembly connected to the housing in the first embodiment and the third configuration showing the jaws in the open position;

Figure 21 is a cross-sectional view of the detachable tool assembly connected to the housing in the first embodiment and the third configuration showing the jaws in the closed position;

Figure 22 is a cross-sectional view of the detachable tool assembly detached from the housing in the first embodiment and the third configuration;

Figure 23 is a perspective view of the third configuration of the coupling between the collet and the housing;

Figure 24 is a cross-sectional view of the housing in the third configuration;

Figure 25 is a perspective view of the detachable tool assembly and the housing in the first embodiment and in a fourth configuration;

Figure 26 is a cross-sectional view of the detachable tool assembly connected to the housing in the first embodiment and the fourth configuration showing the jaws in the open position;

Figure 27 is a cross-sectional view of the detachable tool assembly connected to the housing in the first embodiment and the fourth configuration showing the jaws in the closed position;

Figure 28 is a cross-sectional view of the detachable tool assembly detached from the housing in the first embodiment and the fourth configuration;

Figure 29 is a perspective view of the fourth configuration of the coupling between the collet and the housing;

Figure 30 is a cross-sectional view of the housing in the fourth configuration;

Figure 31 is a perspective view of a second embodiment of the surgical instrument showing the clevis component affixed to the housing;

Figure 32 is a cross-sectional view of the second embodiment showing the detachable tool assembly connected to the housing and the jaws in the open position;

Figure 33 is a cross-sectional view of the second embodiment showing the detachable tool assembly connected to the housing and the jaws in the closed position;

Figure 34 is a cross-sectional view of the detachable tool assembly of the second embodiment showing a pin assembly;

Figure 35 is a perspective view of the clevis component of the second embodiment;

Figure 36 is an exploded perspective view of the detachable tool assembly of the second embodiment;

Figure 37 is a cross-sectional view of a third embodiment of the surgical instrument showing the detachable tool assembly implemented as an electrode;

Figure 38 is a perspective view of the electrode;

Figure 39 is a cross-sectional view of the third embodiment in a first configuration showing the electrode connected to the housing;

Figure 40 is a cross-sectional view of the third embodiment in the first configuration showing the electrode detached from the housing;

Figure 41 is a cross-sectional view of the third embodiment in a second configuration showing the electrode detached from the housing;

Figure 42 is a cross-sectional view of the third embodiment in a third configuration showing the electrode detached from the housing;

Figure 43 is a cross-sectional view of the third embodiment in a fourth configuration showing the electrode detached from the housing; and

Figure 44 is a perspective view of a fourth embodiment of the surgical assembly showing the electrode detached from the housing.

### DETAILED DESCRIPTION OF THE INVENTION

Referring to the Figures, wherein like numerals indicate corresponding parts throughout the several views, a surgical instrument 50 is shown.

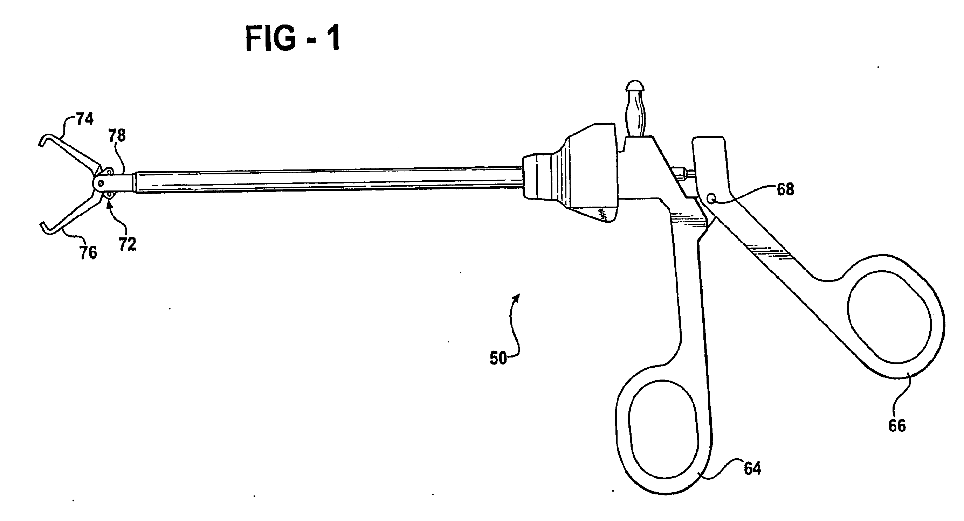

In a first embodiment, as shown in Figure 1, the surgical instrument 50 is forceps primarily for use in performing endoscopic or arthroscopic surgery, specifically for the grasping of objects during such surgery. The components of the surgical instrument 50 are typically formed of a metal, such as a stainless steel. However, the various components of the surgical instrument may be formed of other materials as are known to those skilled in the art.

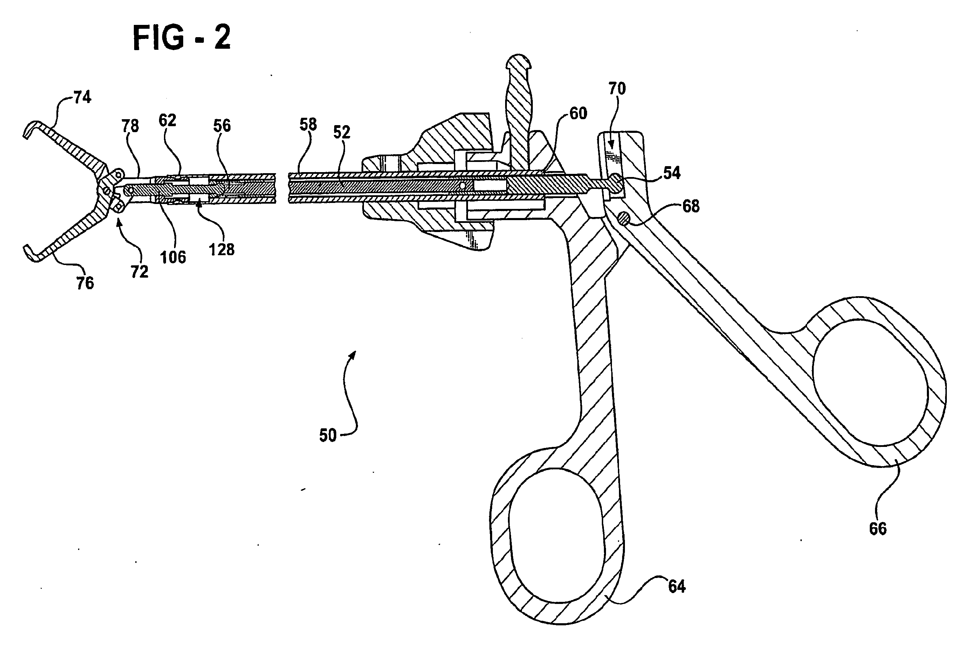

Referring now to Figure 2, the surgical instrument 50 includes a shaft 52 having a proximal end 54 and a distal end 56. Preferably, the shaft 52 is rod-shaped with a generally circular cross section. However, the cross section of the shaft 52 may define other shapes. For instance, the cross section of the shaft 52 may be square or triangular (both not shown).

The shaft 52 is disposed at least partially in a housing 58. That is, the housing 58 surrounds at least a portion of the shaft 52. The housing 58 is preferably tubular-shaped and includes a proximal end 60 and a distal end 62. The housing preferably defines a circular cross section with a hollow center for accommodating the shaft 52. As with the shaft 52, the cross section of the housing 58 may also define other shapes.

At least one handle 64, 66 is operatively connected to the shaft 52, preferably at the proximal end 54 of the shaft 52. In the first embodiment, the surgical instrument 50 includes a fixed handle 64 and a movable handle 66. The fixed handle 64 is attached to the proximal end 60 of the housing 58 and does not move with respect to the housing 58. The movable handle 66 is pivotally connected to the fixed handle 64. A pin 68 disposed through holes (not labeled) in each of the handles 64, 66 provides this pivotable connection.

In the first embodiment, the movable handle 66 is connected to the proximal end 54 of the shaft 52. The proximal end 54 of the shaft 52 defines a ball shape. The movable handle 66 defines a groove 70 for accommodating this ball-shaped proximal end 54. As the movable handle 66 is actuated, the proximal end 54 slides in the groove 66 to longitudinally actuate the shaft 52 within the housing 58. Said another way, the shaft 52 reciprocates, i.e., moves back and forth, within the housing 58 when actuated by the movable handle 66. The movement of the movable handle 66 and the shaft 52 within the housing 58 can be seen at various positions in Figures 2-4 and described in more detail below.

The surgical instrument 50 includes a detachable tool assembly 72. The detachable tool assembly 72 is operatively connected to the shaft 52. The detachable tool assembly 72 includes at least one tool (not numbered). In the first embodiment, the tool is further defined as a first jaw 74 and a second jaw 76 for grasping an object. Those skilled in the art realize numerous design possibilities for the jaws 74, 76 other than that shown in the figures. The detachable tool assembly 72 is easily separable from the shaft 52 and the housing 58 to allow a user of the surgical instrument 50 to quickly change the desired tool. The jaws 74, 76 of the first embodiment are pivotally fastened together such that both jaws 74, 76 are movable as described in detail below. However, in alternative embodiments, one of the jaws 74, 76 could be fixed.

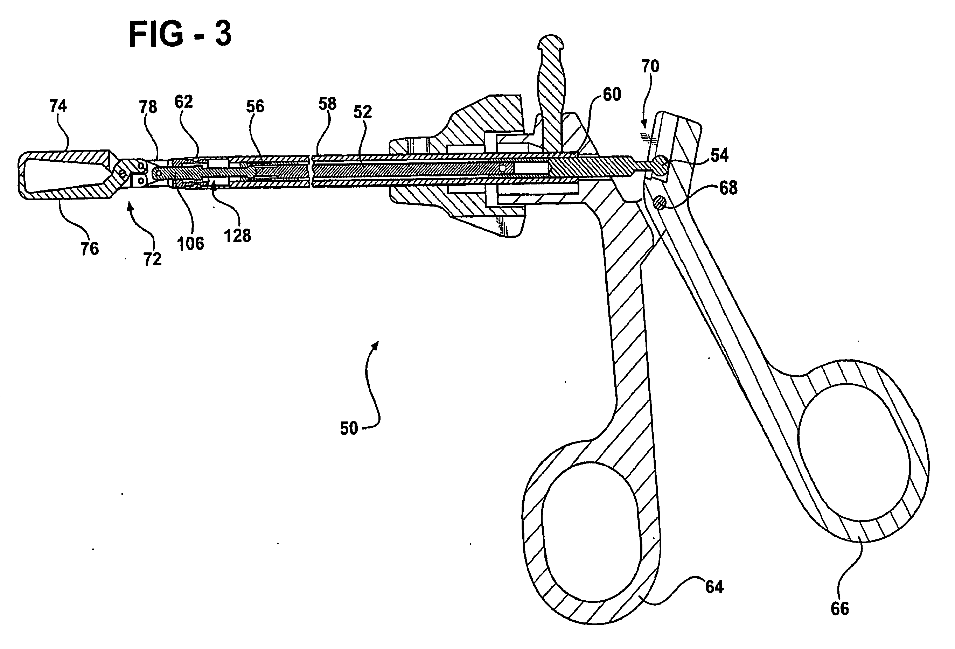

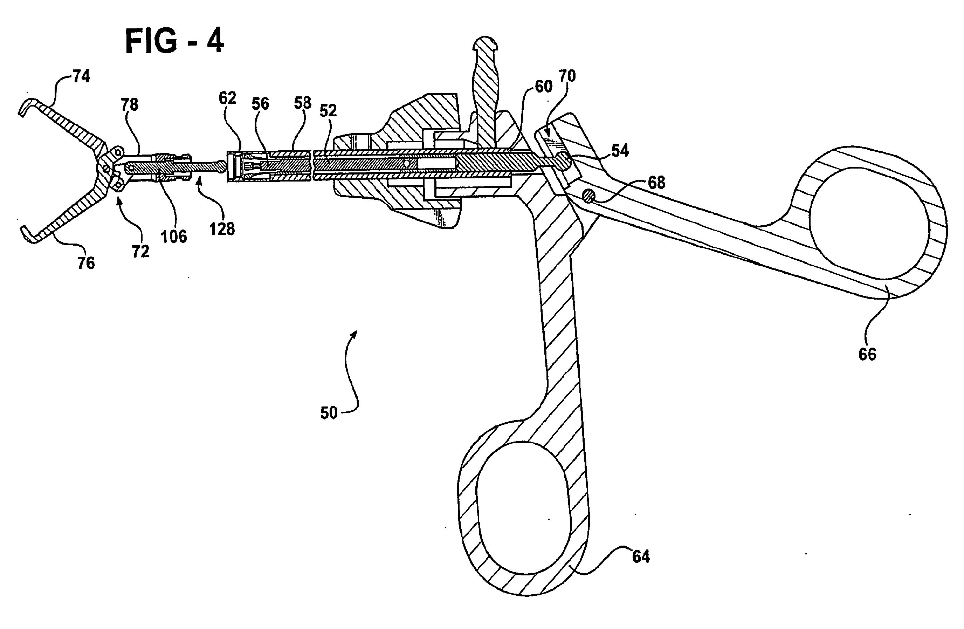

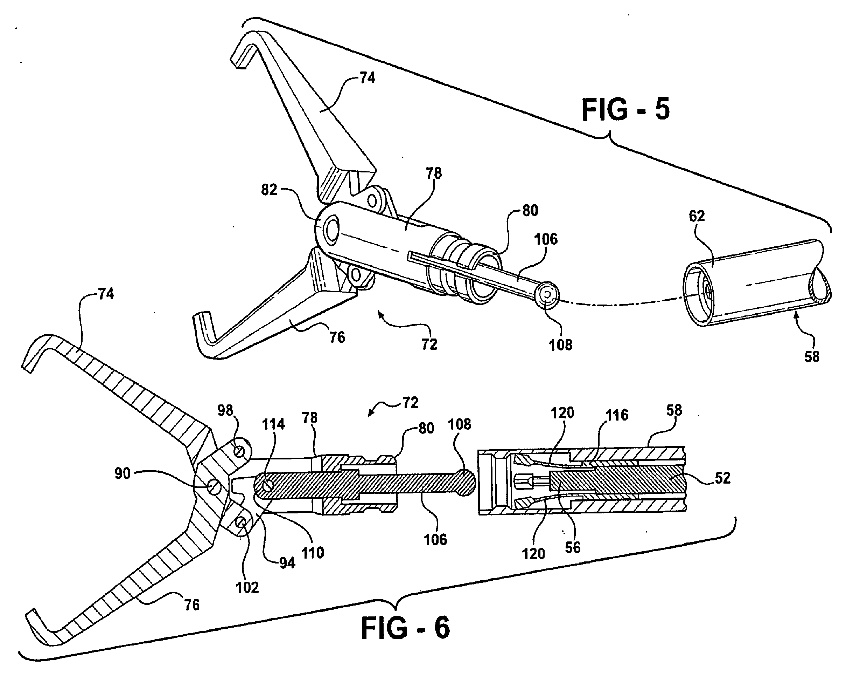

The movable handle 66, shaft 52, and jaws 74, 76 are movable in a plurality of operating positions. These positions include an open position as shown in Figure 2, a closed position as shown in Figure 3, as well as numerous positions in-between. The movable handle 66 and shaft 52 are also movable to an ejected position, where the detachable tool assembly 72 is detached from the instrument 50, as shown in Figure 4. The assembly 72, detached from the housing 58, can also be seen in Figures 5 and 6.

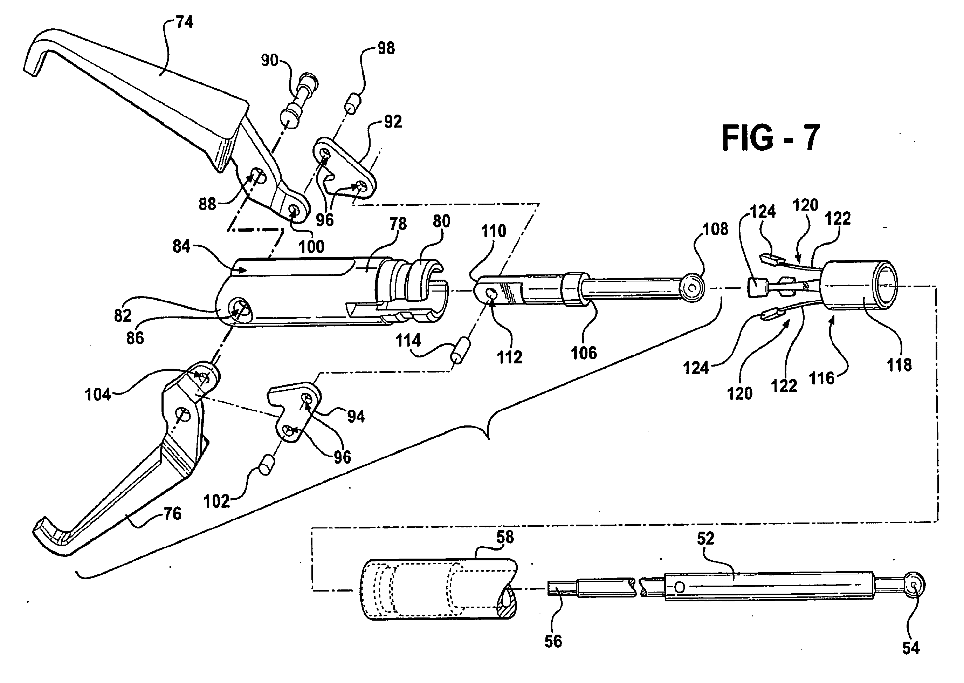

Referring to Figures 5-7, the assembly 72 includes a clevis component 78 having a proximal end 80 and a distal end 82. The proximal end 80 of the clevis component 78 is detachably coupled to the distal end 62 of the housing 58. To mate with the housing 58, the clevis component 78 preferably defines a circular cross section, but other shapes may alternatively be suitable. As best seen in Figure 7, the clevis component 78 component defines a slot 84 extending inward from the distal end 82 to separate a pair of legs (not numbered). A clevis hole 86 is also defined through each leg of the clevis component 78, adjacent the distal end 82. The clevis holes 86 are generally perpendicular to the slot 84.

In the first embodiment, each jaw 74, 76 defines a pivot hole 88. A clevis pin 90 is disposed through the pivot holes 88 and the clevis holes 86 to facilitate pivotal movement of the jaws 74, 76 about the clevis pin 90.

The assembly 72 of the first embodiment also includes a first link 92 and a second link 94. Preferably, the links 92, 94 are at least partially disposed within the slot 84 of the clevis component 78. It is also preferred that two connection holes 96 are defined in each link 92, 94. The first link 92 is connected to the first jaw 74 via a first connection pin 98 disposed through one of the connection holes 96 in the first link 92 and a connection hole 100 in the first jaw 74. The second link 94 is connected to the second jaw 76 via a second connection pin 102 disposed through one of the connection holes 96 in the second link and a connection hole 104 in the second jaw 76.

The assembly 72 of the first embodiment also includes a connection rod 106 having a proximal end 108 and a distal end 110. The proximal end 108 preferably defines a ball shape. A hole 112 is defined adjacent to the distal end 110 of the connection rod 106. A pin 114 connects the hole 112 of the connection rod 108 to one of the connection holes 94 of each of the links 92, 94.

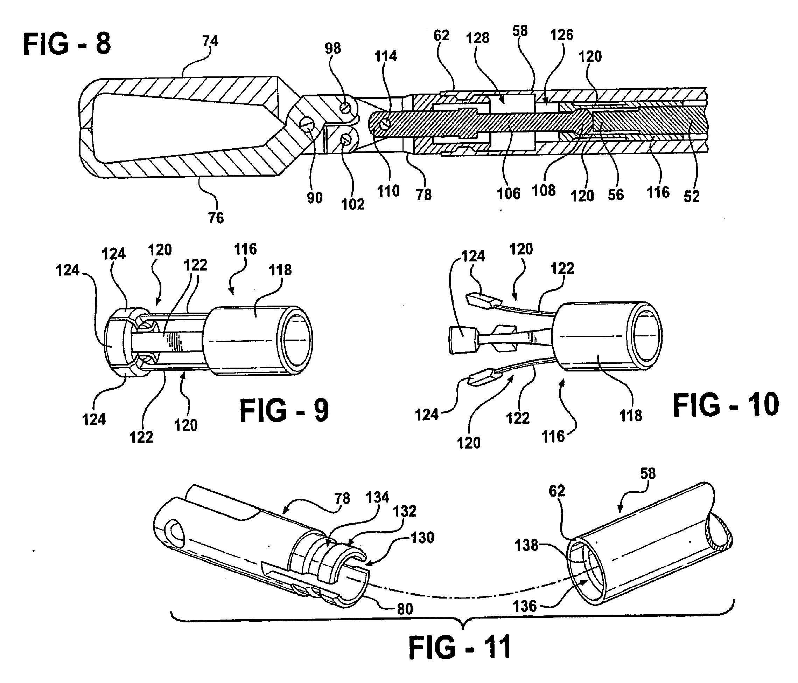

The interconnections described above allow the jaws 74, 76 of the assembly 72 to be movable in response to reciprocating movement of the connection rod 106. As the connection rod 106 is pulled away from the clevis component 78, the jaws 74, 76 move towards one another and into the closed positions, as is shown in Figures 3 and 8. As the connection rod 106 is pushed towards the clevis component 78, the jaws 74, 76 expand away from one another into the open position, as is seen in Figure 2.

As shown in Figures 6-10, the surgical instrument 50 includes a collet 116 for detachably coupling the connection rod 106 to the shaft 52. The collet 116 is disposed within the housing 58. The collet 116 includes a base 118 and a plurality of expandable fingers 120 extending from the base 118. The base 118 of the collet 116 is attached to the distal end of the shaft 52. This attachment of the collet 116 to the shaft 52 may be accomplished by numerous techniques, including, but not limited to, welding, soldering, a fastener, or an adhesive. Those skilled in the art appreciate other techniques to attach the collet 116 to the shaft 52. Alternatively, the shaft 52 and collet 116 may be integrally formed together as a unitary component.

Referring now specifically to Figures 9 and 10, each finger 120 of the collet 116 includes a stem 122 extending from the base and a tip 124 disposed atop of the stem 122. Preferably, each stem 122 is a substantially flat and thin piece of metal. This allows the tips 124 of the fingers 120 to flare apart from one another when not compressed. However, when compressed, the tips 124 of the fingers 120 come together to form a hoop (not numbered). The ball-shaped proximal end 108 of the connection rod 106 is captured between the base 118 and the tips 124 of the fingers 120. Said another way, the collet 116 encompasses the ball-shaped proximal end 108. The connection rod 106, except for the ball-shaped proximal end 108, extends through a center (not numbered) of the hoop when the ball-shaped proximal end 108 is captured by the collet 116.

Referring to Figures 6 and 8, the housing 58 preferably defines a central opening 126 and a cavity 128. The cavity 128 is disposed adjacent the distal end 62 of the housing 58. The cavity 128 has a cross-sectional width greater than a cross-sectional width of the central opening 126. In the preferred embodiment, where the housing 58 has a circular cross section, the cavity 128 has a diameter that is greater than a diameter of the central opening 126.

Since the base of the collet 116 is attached to the distal end 56 of the shaft 52, the collet 116 moves within the housing as the shaft 52 reciprocates based on motion of the movable handle 66. The collet 116 is movable between the ejected position and the operating positions. In the operating positions, the fingers 120 of the collet 116 are substantially disposed within the central opening 126, such that the fingers 120 are collapsed to capture the ball-shaped proximal end 108 of the connection rod 106. In the ejected position, the fingers 120 of the collet 116 are substantially disposed within the cavity 128. As such, the fingers 120 flare away from one another in the cavity 128 to release the connection rod 106. Therefore, as the movable handle 66 is moved away from the fixed handle 64, the collet 116 moves into the ejected position, where the detachable tool assembly 72 can be removed from the surgical instrument 50. Even in the ejected position, the fingers 120 of the collet 116 are disposed within the housing. Thus, the fingers 120 are protected from accidental damage and breakage.

In addition to the coupling provided by the collet 116 and the connection rod 106, a secondary coupling is provided by the clevis component 78 and the housing 58. Specifically, the proximal end 80 of the clevis component 78 is removably coupled to the distal end 62 of the housing 58. As the movable handle 66 forces the shaft 62 in the ejected position, the shaft 62 forces the decoupling of the clevis component 78 from the housing 58. Thus, the surgical instrument 50 provides two separate couplings that are both controlled by the one-handed operation of the movable handle 66. This provides an extremely stable connection between the detachable tool assembly 72 and the housing 58.

The present invention discloses numerous configurations for achieving this secondary coupling. A first configuration of the secondary coupling is shown in Figures 5-8 and 11-12. Referring to Figure 11, the clevis component 78 defines a pair of slits 130 protruding inward from the proximal end 80. The proximal end 80 of the clevis component 78 also defines a recessed outer surface 132 with a groove 134 indented into the recessed outer surface 132. The distal end 62 of the housing 58 defines an inner surface 136 with a tongue 138 protruding inward. The tongue 138 of the housing 58 and the groove 134 of the clevis component 78 mate to couple the housing 58 and clevis component 78 together. The slit 130 allows the proximal end 80 of the clevis component 78 to compress, thus allowing connection and disconnection of the clevis component 78 from the housing 58.

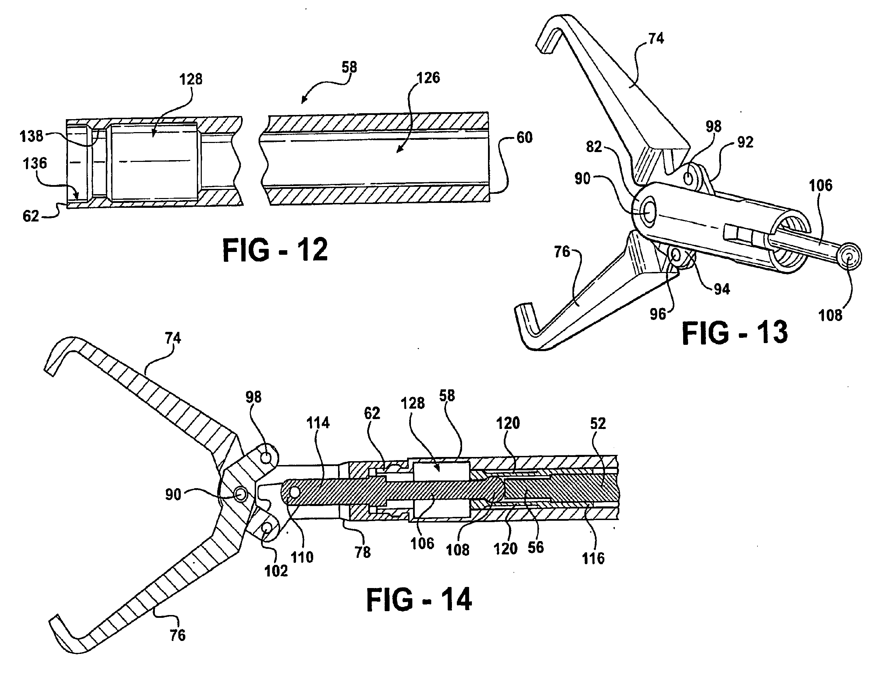

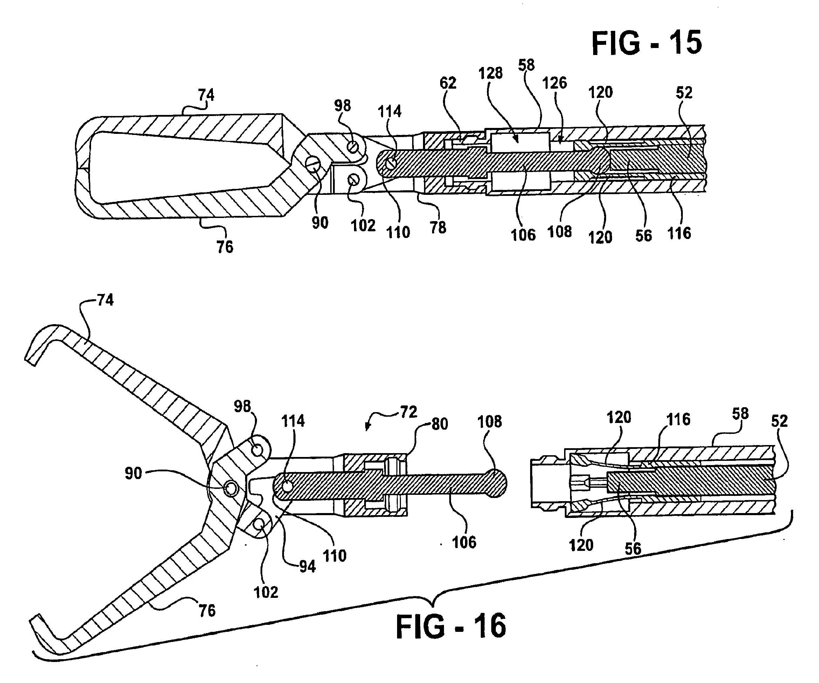

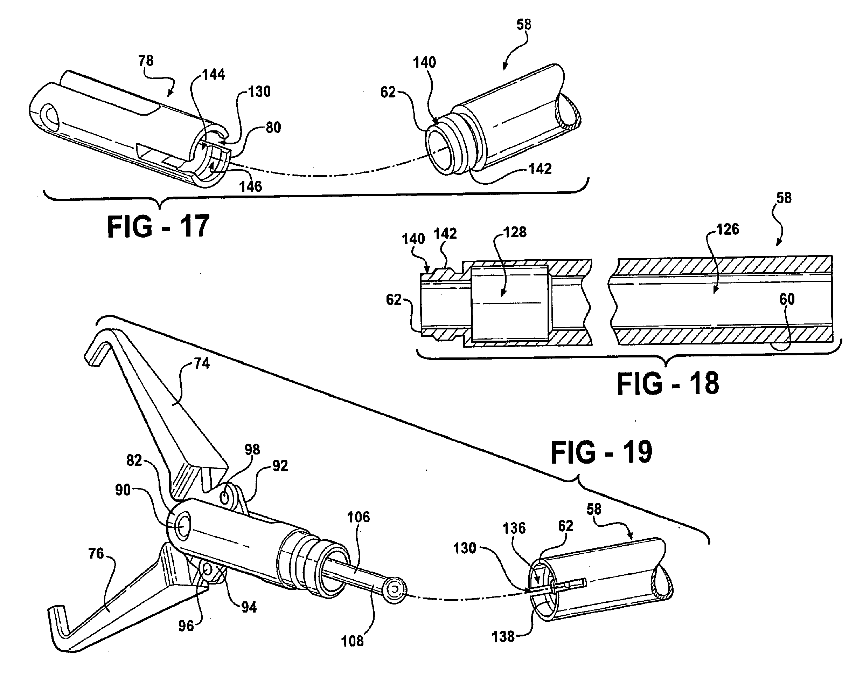

A second configuration of the secondary coupling is shown in Figures 13-18. Referring specifically to Figure 17 the distal end 62 of the housing 58 defines a recessed outer surface 140 with a tongue 142 projecting outward from the outer surface 140. The clevis component 78 defines an inner surface 144 defining a groove 146 for mating with the tongue 142. The slit 130 protrudes inward from the proximal end 80 of the clevis component 78.

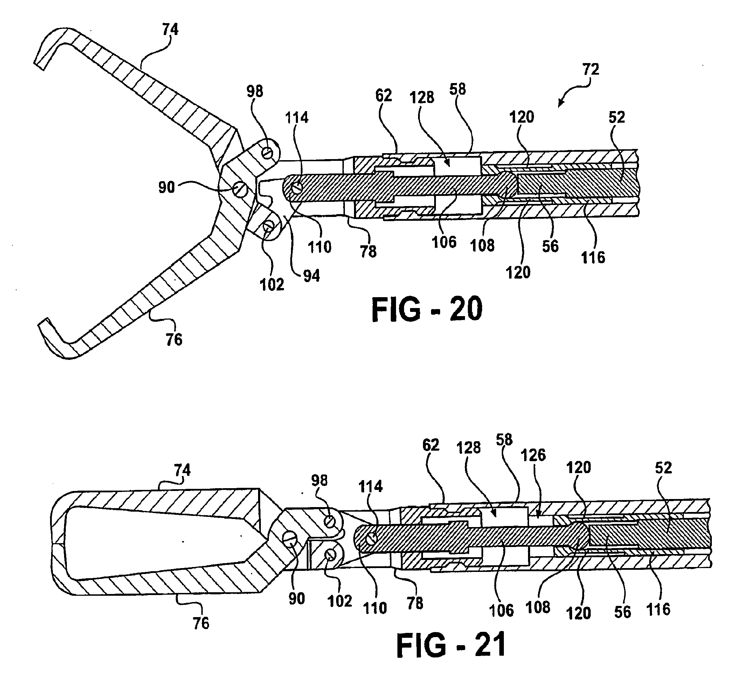

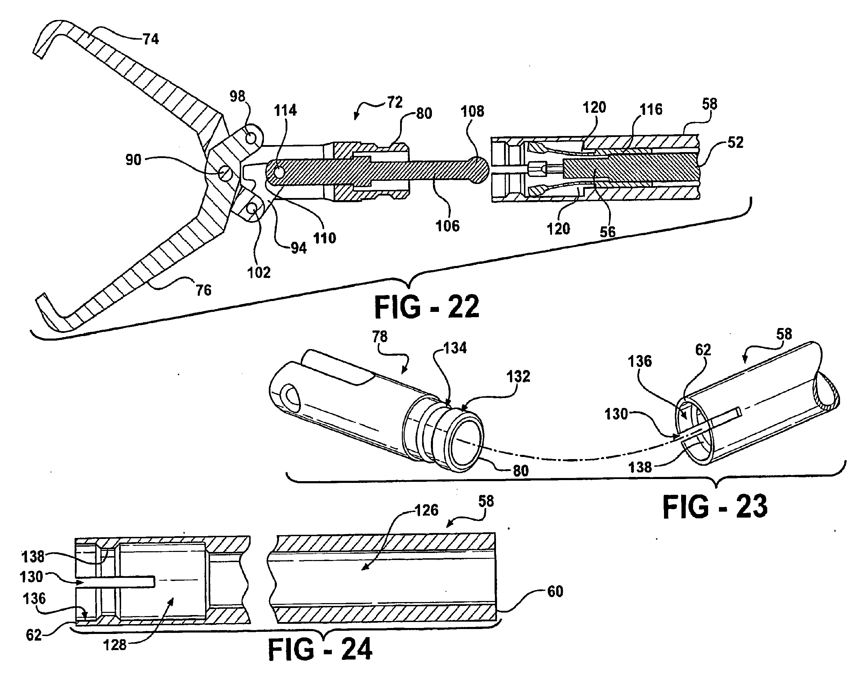

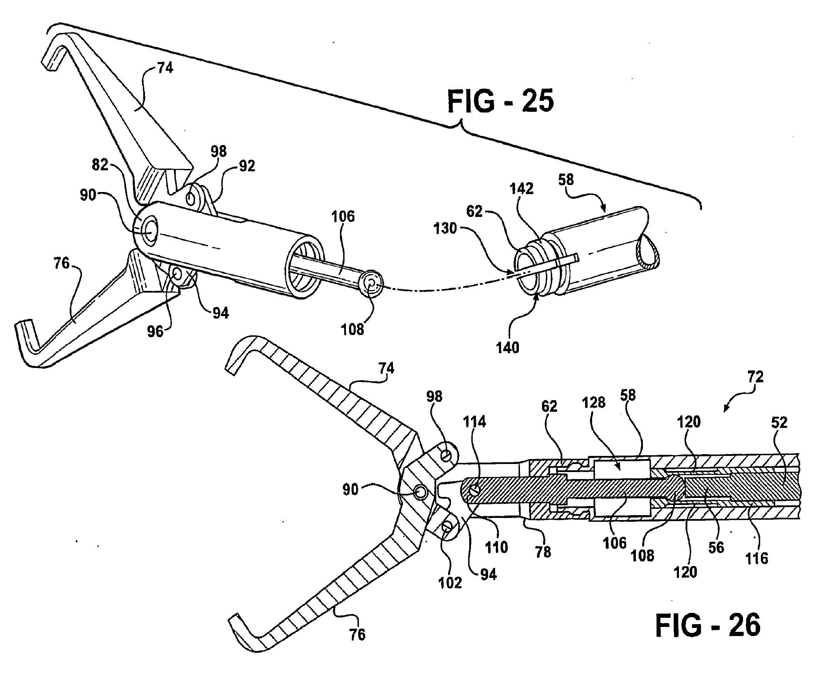

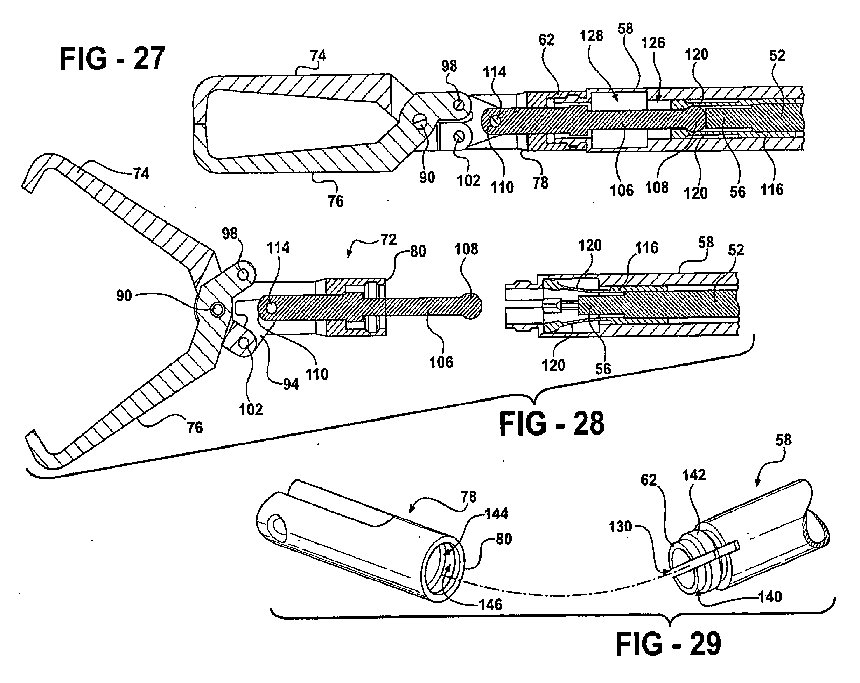

Figures 19-24 show a third configuration of the secondary coupling. The third configuration is substantially identical to the first configuration, except that the slit 130 is defined by the housing 58 and projects inward from the distal end 62. A fourth configuration, shown in Figures 25-30, is substantially identical to the second configuration, except that the slit 130 is defined by the housing 58 and projects inward form the distal end 62.

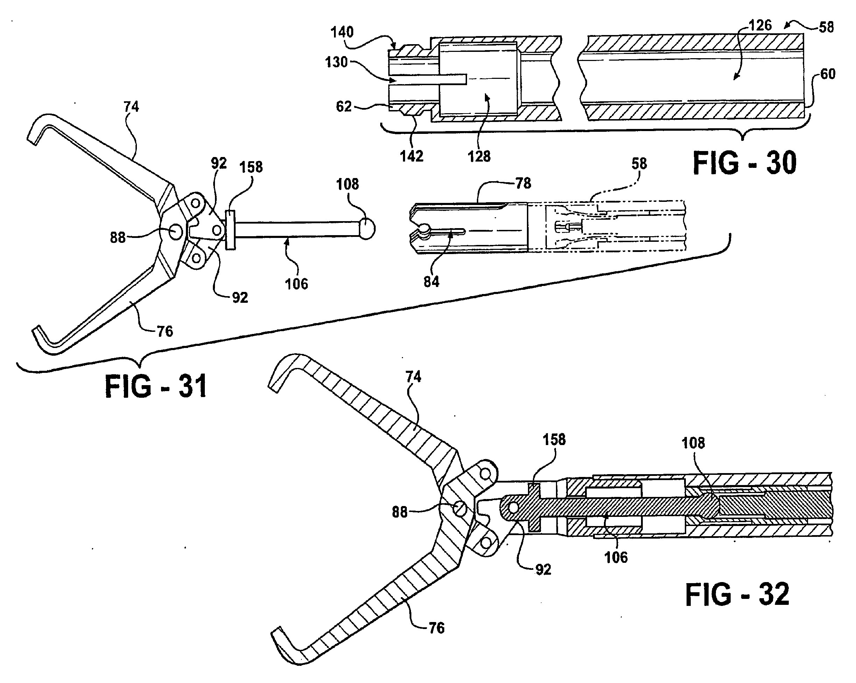

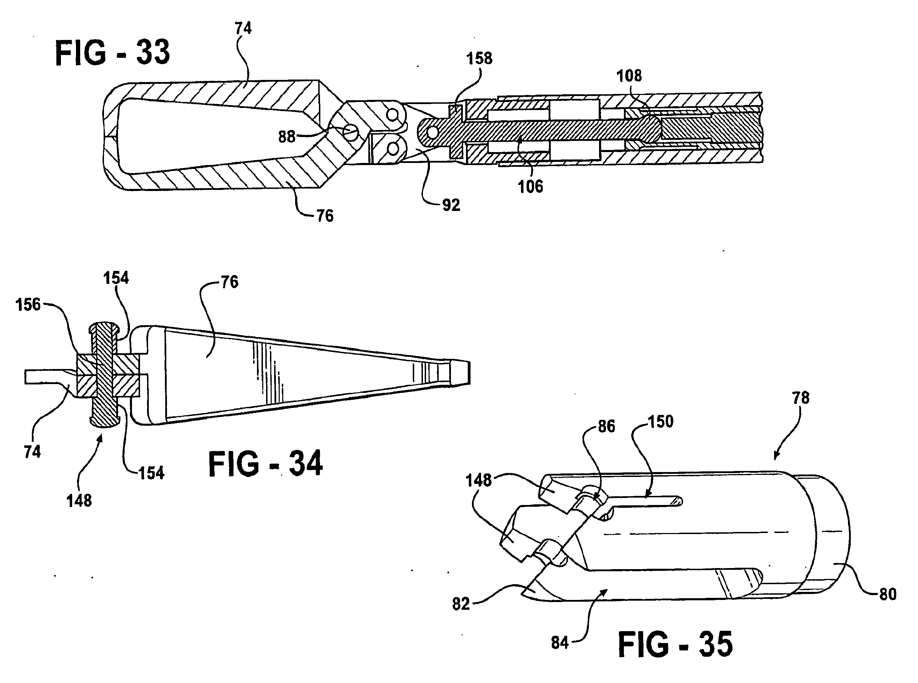

In a second embodiment of the invention, as shown in Figures 31-35, the clevis component 78 is affixed to the housing 58. This differs from the first embodiment, where the clevis component 78 is part of the detachable tool assembly 72. Referring to Figure 33, the proximal end 80 of the clevis component 78 is recessed to mate with the distal end 62 of the housing 58. In the second embodiment, the distal end 82 of the clevis component 78 includes tapered inlets 148. These tapered inlets 148 define a pair of inlet slots 149 extending inward to each clevis hole 86. A slit 150 is also defined projecting inward from each clevis hole 86 to allow the distal end 82 of the clevis component 78 to flex.

In the second embodiment, a pin assembly 152 pivotally connects the jaws 74, 76 via the pivot holes 88. The pin assembly 152 includes a pair of expanded heads 154 interconnected by a pin 156. The pin assembly 152, and specifically the expanded heads 154, extends beyond the jaws 74, 76 on both sides of the jaws 74, 76. To couple the assembly 72 with the housing 58, the pin assembly 152 is slid through the inlet slots 149 and into the clevis holes 86. The expanded heads 154 snap lock with the cleivs component 78 in the clevis holes 86. The connection rod 106 carries a perpendicular extension 158. The perpendicular extension 158 mates with the slot 84 of the clevis component 78 which force correct alignment of the assembly 72 and particularly the expandable heads 154 to the clevis holes 86.

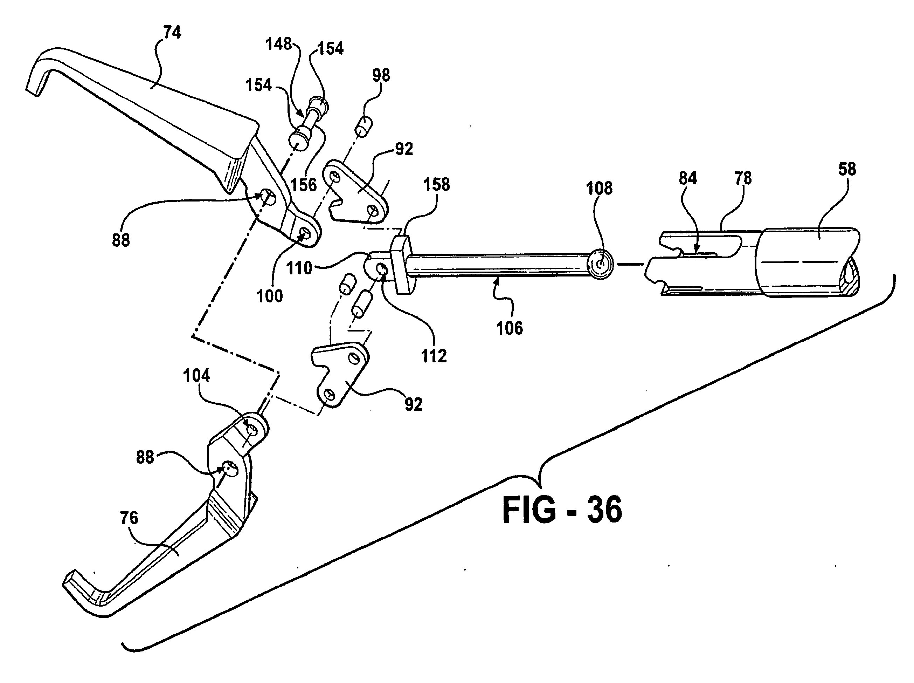

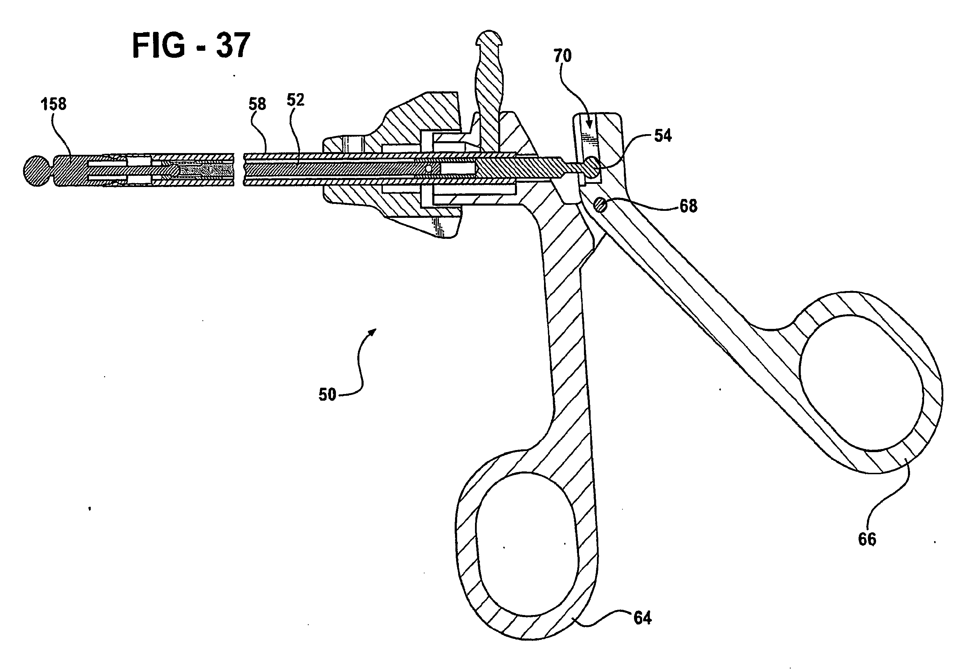

Figures 35-41 show a third embodiment of the invention. In the third embodiment the detachable tool assembly 72 is implemented as an electrode 158 for applying electrical current to tissue during a surgical procedure. The electrode 158 is preferably formed as a unitary part, i.e., its components are not fastened together. However, those skilled in the art realize numerous techniques to form the electrode 158.

The electrode 158 includes a proximal end 160 and a distal end 162. In the fourth embodiment, as illustrated, the distal end 162 is ball shaped to provide a round surface for application of electrical energy. However, those skilled in the art realize other shapes for the distal end 162, such as, but not limited to, a needle tip or a scalpel shape.

The electrode 158 forms a void 163 extending inward from the proximal end 160. The electrode 158 includes a connection rod 164 that extends through the void and outward past the proximal end 160. The connection rod 164 includes a ball-shaped end 166. The ball-shaped end mates with the collet 116 such that the electrode 158 may be secured in place or ejected by the fingers 120 of the collet 116.

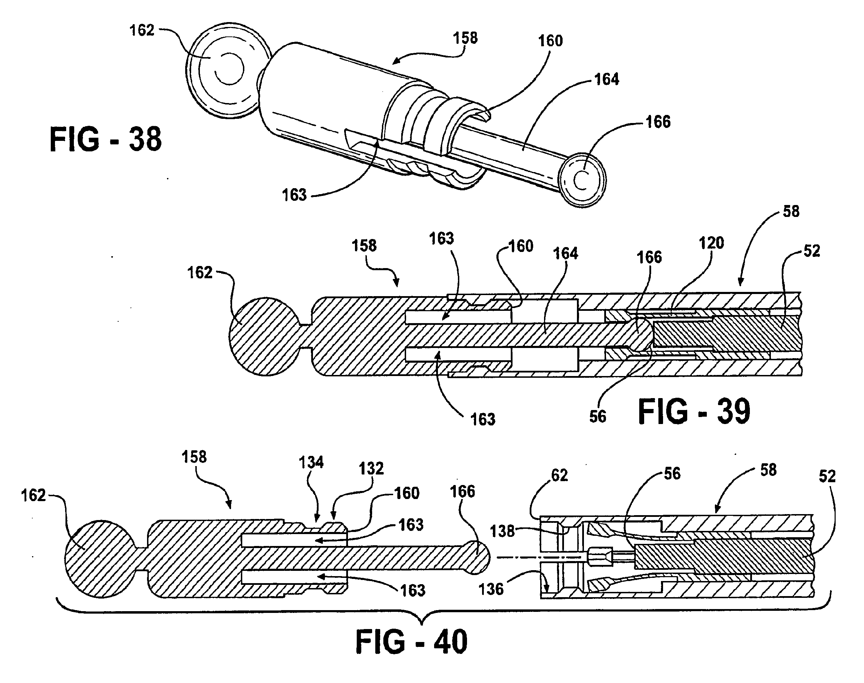

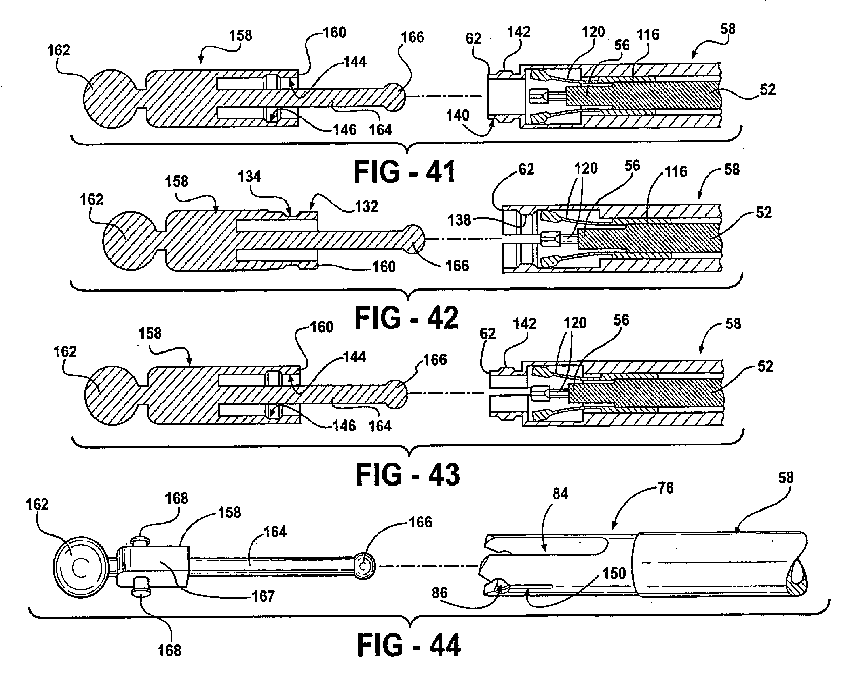

The proximal end 160 of the electrode 158 is detachably connected to the distal end 62 of the housing 58 in a similar fashion to the collet component 78 and housing 58 in the first embodiment. In a first configuration, as shown in Figures 38-40, the proximal end 160 of the electrode 158 defines the recessed outer surface 132 with the groove 134. The housing 58 defines the inner surface 136 with the tongue 138 for matting with the groove 134. A second configuration is shown in Figure 41. In the second configuration, the electrode 158 defines an inner surface 144 with the groove 146 adjacent the proximal end 160. The housing 58 defines the recessed outer surface 140 with the tongue 142 adjacent the distal end 62. A third configuration, shown in Figure 41, is similar to the first configuration, except the pair of slits 130 are defined in the housing 58 extending inward from the distal end 62. A fourth configuration, shown in Figure 42, is similar to the second configuration, except a pair of slits 130 are defined in the housing 58 extending inward from the distal end 62.

A fourth embodiment of the surgical instrument 50 is shown in Figure 44. In this fourth embodiment, the detachable tool assembly is implemented as the electrode 158. The electrode 158 includes a base portion 167. The distal end 162 and the connection rod 162 extend in opposite directions from the base portion 167. A pair of expanded heads 168 extends from the base portion 167 and each head 168 is generally perpendicular to the connection rod 164. The clevis component 78 is attached to the housing 58 and configured as in the second embodiment. Thus, the expanded heads 168 of the electrode 158 snap lock with the clevis holes 86 in the clevis component 78 when attached to the housing 58. In the ejected position, the shaft 52 pushes the electrode 158 away from the housing 58 and forces disengagement of the expanded heads 168 from the clevis holes 86.

The electrode 158 is preferably electrically connected to a power supply (not shown) which provides the electrical current. The electrical current is preferably supplied via the shaft 52 and the connection rod 164. Certain components of the instrument 50, such as the housing 58 and the handles 64, 66, are preferably electrically insulated from the housing 58 and the electrode 158. This electrical insulation may be accomplished by coating the metallic components with an insulating material, such as rubber, or forming the components out of an insulating material, such as plastic.

Other embodiments of the detachable tool assembly 72 may be configured for use with the surgical instrument 50. For instance, the detachable tool assembly 72 may include a pair of blades (not shown) to act as surgical scissors. The detachable tool assembly 72 could also include a single scalpel blade (not shown). Those skilled in the art will realize implementations of the detachable tool assembly.

The present invention has been described herein in an illustrative manner, and it is to be understood that the terminology which has been used is intended to be in the nature of words of description rather than of limitation. Many modifications and variations of the invention are possible in light of the above teachings. The invention may be practiced otherwise within the scope of the appended claims.

## Claims

1. A surgical instrument (50) comprising:
a shaft (52) having a proximal end (54) and a distal end (56);
a housing (58) surrounding at least a portion of said shaft (52) and defining a central opening (126) and a cavity (128) wherein said cavity (128) has a cross-sectional width greater than a cross-sectional width of said central opening (126);
at least one handle (64, 66) operatively connected to said proximal end (54) of said shaft (52) for longitudinally actuating said shaft (52) within said housing (58);
a detachable tool assembly (72) including a connection rod (106) having a proximal end (108) and a distal end (110) and at least one tool operatively connected to said connection rod (106); and
a collet (116) disposed within said housing (58) and detachably coupling said shaft (52) and said connection rod (106);
said collet (116) including a base (118) attached to said distal end of said shaft (52) and a plurality of expandable fingers (120) extending from said base (118) such that said fingers (120) capture said proximal end of said connection rod (106) when in a collapsed position and release said proximal end of said connection rod (106) when in an expanded position; and
said collet (116) is moveable within said housing (58) between an ejected position and at least one operating position as said shaft (52) moves longitudinally through said housing (58) wherein said fingers (120) are collapsed within said central opening (126) and capture said ball-shaped proximal end (108) of said connection rod (108) in said at least one operating position and said fingers (120) flare away from one another in said cavity (128) to release said connection rod (106) in said ejected position.

2. A surgical instrument (50) as set forth in claim 1 wherein each of said fingers (120) includes a stem (122) extending from said base (118) and a tip (124) disposed atop said stem (122).

3. A surgical instrument (50) as set forth in claim 2 wherein said tips (124) of said fingers (120) form a hoop when said collet (116) is in said collapsed position for accommodating said connection rod (106) through said hoop but retaining said proximal end of said connection rod (106) between said tips (124) and said base (118).

4. A surgical instrument (50) as set forth in claim 1 wherein said at least one tool is further defined as a pair of jaws (74, 76) movable between an open position and a closed position.

5. A surgical instrument (50) as set forth in claim 4 wherein said tool assembly (72) further includes a clevis component (78) defining a hole (86, 88) and detachably coupled to said distal end of said housing (58),

6. A surgical instrument (50) as set forth in claim 5 wherein said tool assembly (72) further includes a clevis pin (90, 152) pivotally fastening said jaws (74, 76) to said clevis component (78) via said hole (86, 88).

7. A surgical instrument (50) as set forth in claim 6 wherein said tool assembly (72) includes a pair of links (92, 94) operatively connecting said connection rod (106) to said jaws (74, 76) such that said jaws (74, 76) move between said open and closed positions in response to movement of said handle (64, 66).

8. A surgical instrument (50) as set forth in claim 5 wherein said clevis component (78) defines a groove (134, 146) and said housing (58) includes a tongue (138, 142) for mating with said groove (134, 146) to detachably couple said clevis component (78) with said housing (58).

9. A surgical instrument (50) as set forth in claim 5 wherein said devis component (78) includes a tongue and said housing (58) defines a groove for mating with said tongue to detachably couple said clevis component (78) with said housing (58).

10. A surgical instrument (50) as set forth in claim 1 wherein said at least one tool is further defined as an electrode (158).

11. A surgical instrument (50) as claimed in any one of claims 1 to 10 further comprising
a clevis component (78); and
a second coupling formed by said clevis component (78) and said housing (58) for detachably coupling said assembly (72) to said housing (58).

## Patentansprüche

1. Chirurgisches Instrument (50), umfassend:
einen Schaft (52) mit einem proximalen Ende (54) und einem distalen Ende (55);
ein Gehäuse (58), das wenigstens einen Abschnitt des Schafts (52) umgibt und eine zentrale Öffnung (126) sowie eine Kavität (128) definiert, wobei die Kavität (128) eine Querschnittsbreite aufweist, die größer als eine Querschnittsbreite der zentralen Öffnung (126) ist;
wenigstens einen Griff (64, 66), der wirksam mit dem proximalen Ende (54) des Schafts (52) verbunden ist, um den Schaft (52) in dem Gehäuse (58) longitudinal zu betätigen;
eine abnehmbare Werkzeuganordnung (72), die eine Verbindungsstange (106) mit einem proximalen Ende (108) und einem distalen Ende (110) sowie wenigstens ein Werkzeug umfasst, das wirksam mit der Verbindungsstange (106) verbunden ist; und
eine Spannhülse (116), die in dem Gehäuse (58) angeordnet ist und den Schaft (52) und die Verbindungsstange (106) abnehmbar verbindet;
wobei die Spannhülse (116) eine Basis (118) umfasst, die an das distale Ende des Schafts (52) angebracht ist, sowie eine Vielzahl von ausdehnbaren Fingern (120), die sich von der Basis (118) derart ausdehnen, dass die Finger (120) das proximale Ende der Verbindungsstange (106) in Eingriff nehmen, wenn diese sich in einer eingezogenen Position befinden, und das proximale Ende der Verbindungsstange (106) freigeben, wenn sich diese in einer ausgedehnten Position befinden; und
die Spannhülse (116) in dem Gehäuse (58) zwischen einer ausgefahrenen Position und wenigstens einer Betriebsposition bewegt werden kann, wenn sich der Schaft (52) longitudinal durch das Gehäuse (58) bewegt, wobei die Finger (120) in der zentralen Öffnung (126) eingezogen sind und das ballförmige proximale Ende (108) der Verbindungsstange (106) in der wenigstens einen Betriebsposition in Eingriff nehmen und die Finger (120) in der Kavität (128) voneinander weg verlaufen, um die Verbindungstange (106) in der ausgefahrenen Position freizugeben.

2. Chirurgisches Instrument (50) nach Anspruch 1, wobei jeder der Finger (120) einen Stiel (122) umfasst, der sich von der Basis (118) ausdehnt, sowie eine Spitze (124), die am oberen Ende des Stiels (122) angeordnet ist.

3. Chirurgisches Instrument (50) nach Anspruch 2, wobei die Spitzen (124) der Finger (120) einen Reifen ausbilden, wenn sich die Spannhülse (116) in der eingezogenen Position befindet, um die Verbindungsstange (106) durch den Reifen aufzunehmen, aber das proximale Ende der Verbindungsstange (106) zwischen den Spitzen (124) und der Basis (118) zurückzuhalten.

4. Chirurgisches Instrument (50) nach Anspruch 1, wobei das wenigstens eine Werkzeug ferner als ein Zangenpaar (74, 76) definiert ist, die zwischen einer offenen Position und einer geschlossenen Position bewegt werden können.

5. Chirurgisches Instrument (50) nach Anspruch 4, wobei die Werkzeuganordnung (72) ferner eine Gelenkkomponente (78) umfasst, die ein Loch (86, 88) definiert und abnehmbar mit dem distalen Ende des Gehäuses (58) verbunden ist.

6. Chirurgisches Instrument (50) nach Anspruch 5, wobei die Werkzeuganordnung (72) ferner einen Gelenkbolzen (90, 152) umfasst, der die Zangen (74, 76) über das Loch (86, 88) schwenkbar an die Gelenkkomponente (78) befestigt.

7. Chirurgisches Instrument (50) nach Anspruch 6, wobei die Werkzeuganordnung (72) ein Paar von Verbindungselementen (92, 94) umfasst, die wirksam die Verbindungsstange (106) mit den Zangen (74, 76) derart verbindet, dass sich die Zangen (74, 76) in Reaktion auf eine Bewegung des Griffs (64, 66) zwischen der offenen und der geschlossenen Position bewegen.

8. Chirurgisches Instrument (50) nach Anspruch 5, wobei die Gelenkkomponente (78) eine Nut (134, 146) definiert und das Gehäuse (58) eine Feder (138, 142) für das Zusammenwirken mit der Nut (134, 146) umfasst, um die Gelenkkomponente (78) abnehmbar mit dem Gehäuse (58) zu verbinden.

9. Chirurgisches Instrument (50) nach Anspruch 5, wobei die Gelenkkomponente (78) eine Feder umfasst und das Gehäuse (58) eine Nut für das Zusammenwirken mit der Feder definiert, um die Gelenkkomponente (78) abnehmbar mit dem Gehäuse (58) zu verbinden.

10. Chirurgisches Instrument (50) nach Anspruch 1, wobei das wenigstens eine Werkzeug ferner als eine Elektrode (158) definiert ist.

11. Chirurgisches Instrument (50) nach einem der Ansprüche 1 bis 10, ferner umfassend:
eine Gelenkkomponente (78); und
eine zweite Verbindung, die von der Gelenkkomponente (78) und dem Gehäuse (58) ausgebildet wird, um die Anordnung (72) abnehmbar mit dem Gehäuse (58) zu verbinden.

## Revendications

1. Instrument chirurgical (50) comprenant :
- une tige (52) présentant une extrémité proximale (54) et une extrémité distale (56) ;
- un boîtier (58) entourant au moins une partie de ladite tige (52) et définissant une ouverture centrale (126) et une cavité (128), dans lequel ladite cavité (128) présente une section transversale supérieure à une section transversale de ladite ouverture centrale (126) ;
- au moins une poignée (64, 66) connectée de manière opérationnelle à ladite extrémité proximale (54) de ladite tige (52) permettant d'actionner de manière longitudinale ladite tige (52) à l'intérieur dudit boîtier (58) ;
- un ensemble à outil amovible (72) comprenant une tige de connexion (106) présentant une extrémité proximale (108) et une extrémité distale (110), et au moins un outil connecté de manière opérationnelle à ladite tige de connexion (106); et
- un mandrin (116) disposé à l'intérieur du boîtier (58) et couplant, de manière amovible, ladite tige (52) et ladite tige de connexion (106) ;
- ledit mandrin (116) comprenant une base (118) attaché à ladite extrémité distale de ladite tige (52) et une pluralité de doigts expansibles (120) s'étendant depuis ladite base (118) de telle sorte que les doigts (120) capturent ladite extrémité proximale de ladite tige de connexion (106) dans une position rabattue et libèrent ladite extrémité proximale de ladite tige de connexion (106) dans une position expansée ; et
- ledit mandrin est déplaçable à l'intérieur dudit boîtier (58) entre une position éjectée et au moins une position opérationnelle alors que ladite tige (52) se déplace de manière longitudinale à l'intérieur dudit boîtier (58), et dans lequel lesdits doigts (120) sont rabattus à l'intérieur de l'ouverture centrale (126) et capturent ladite extrémité proximale en forme de boule (108) de ladite tige de connexion (106), dans la au moins une position opérationnelle, et lesdits doigts (120) s'étendent radicalement les uns des autres à l'intérieur de ladite cavité (128), afin de libérer ladite tige de connexion (106), dans la position éjectée.

2. Instrument chirurgical (50) selon la revendication 1, dans lequel chacun desdits doigts (120) comprend une tige (122) s'étendant depuis la base (118) et une pointe (124) disposée en haut de ladite tige (122).

3. Instrument chirurgical (50) selon la revendication 2, dans lequel lesdites pointes (124) desdits doigts (120) forment un anneau lorsque ledit mandrin (116) est dans une position rabattue, ladite tige de connexion (106) passant à travers l'anneau, et le mandrin retenant ladite extrémité proximale de ladite tige de connexion {106} entre lesdites pointes (124) et ladite base (118).

4. Instrument chirurgical (50) selon la revendication 1, dans lequel ledit au moins un outil est en outre défini comme étant une paire de mâchoires (74, 76) déplaçable entre une position ouverte et un position fermée.

5. Instrument chirurgical (50) selon la revendication 4, dans lequel ledit ensemble à outil (72) comprend en outre un composant à manille qui définit une ouverture (86, 88), couplé de manière amovible à ladite extrémité distale dudit boîtier (58).

6. Instrument chirurgical selon la revendication 5, dans lequel ledit ensemble à outil (72) comprend en outre une goupille (90, 152) fermant de manière pivotante lesdites mâchoires (74, 76) audit composant à manille (78) via ladite ouverture (86, 88).

7. Instrument chirurgical selon la revendication 6, dans lequel ledit ensemble à outil (72) comprend une paire de maillons (92, 94) connectant de manière opérationnelle ladite tige de connexion (106) auxdites mâchoires (74, 76) de telle sorte que celles-ci se déplacent entre lesdites positions ouverte et fermée en réponse à un mouvement de ladite poignée (64, 66).

8. Instrument chirurgical (50) selon la revendication 5, dans lequel ledit composant à manille (78) définit une rainure (134, 146) et ledit boîtier comprend une languette (138, 142) pouvant s'accoupler avec ladite rainure (134, 146), afin de coupler de manière amovible ledit composant à manille (78) avec ledit boîtier (58).

9. Instrument chirurgical (50) selon la revendication 5, dans lequel ledit composant à manille (78) comprend une languette et ledit boîtier (58) comprend une rainure pouvant s'accoupler avec ladite languette, afin de coupler de manière amovible ledit composant à manille (78) avec ledit boîtier (58).

10. Instrument chirurgical (50) selon la revendication 1, dans lequel ledit au moins un outil est en outre défini en tant qu'électrode (158).

11. Instrument chirurgical (50) selon l'une quelconque des revendications 1 à 10, comprenant en outre :
- un composant à manille (78) ; et
- un deuxième couplage formé par ledit composant à manille (78) et ledit boîtier (58) afin de coupler de manière amovible ledit ensemble (72) audit boîtier (58).
